# EUROPEAN PATENT APPLICATION

(11) **EP 3 025 710 A1**
(43) Date of publication of application: **01.06.2016**
(21) Application number: 15200814.0
(22) Date of filing: 02.03.2012
(51) Int. Cl.: A61K 31/137, A61K 9/00, A61K 45/06, A61K 47/12

(54) **PARENTERAL ADMINISTRATION OF TAPENTADOL**

(30) Priority: 04.03.2011 US 201161449317 P; 03.05.2011 EP 11003602
(62) Divisional of application: 12709792.1
(71) Applicant: Grünenthal GmbH, 52078 Aachen (DE)
(72) Inventor: Reinhold, Ulrich, 52076 Aachen (DE); Schiller, Marc, 52076 Aachen (DE); Wulsten, Eva, 47877 Willich (DE); Christoph, Thomas, 52080 Aachen (DE); Bloms-Funke, Petra, 52146 Würselen (DE); Schiene, Klaus, 41363 Jüchen (DE); Inghelbrecht, Sabine Karine Katrien, 2340 Beerse (BE); Embrechts, Roger Carolus Augusta, 2340 Beerse (BE); Feil, Ulrich, 63739 Aschaffenburg (DE)
(74) Representative: Kutzenberger Wolff & Partner

(57) **Abstract**

The invention relates to an aqueous pharmaceutical composition adapted for parenteral administration of tapentadol or a physiologically acceptable salt thereof having a pH value of at least 5.4.

## Description

The invention relates to an aqueous pharmaceutical composition adapted for parenteral administration of tapentadol or a physiologically acceptable salt thereof having a pH value of at least 4.0, preferably of at least 5.4.

Tapentadol is a centrally-acting analgesic with a dual mode of action as an agonist at the µ-opioid receptor and as a norepinephrine reuptake inhibitor (cf. T.M. Tzschentke et al., Drugs of the future, 2006, 12, 1053-1061). In humans, the affinity of tapentadol to the recombinantly produced µ-opioid receptor is 18-times less than that of morphine. However, clinical studies have shown the pain-alleviating action of tapentadol to be only two to three times less than that of morphine. The only slightly reduced analgesic efficacy with a simultaneously 18-times reduced affinity to the recombinant µ-opioid receptor indicates that the noradrenaline transporter inhibiting property of tapentadol also contributes to its analgesic efficacy. Consequently, it may be assumed that tapentadol has a similar analgesic efficacy to that of pure µ-opioid receptor agonists but has fewer of the side effects associated with the µ-opioid receptor. The compound can be used in the form of its free base or as a salt or solvate. The production of the free base is known for example from EP-A 693 475. Dosage forms of tapentadol are known from the prior art, e.g. WO 02/67651, WO 03/035053, WO 2006/002886, WO 2007/128412, WO 2007/128413, WO 2008/110323, WO 2009/067703, WO 2009/092601, and US2010-272815.

However, those known dosage forms containing tapentadol are not satisfactory in every respect and there is a demand of pharmaceutical formulations which have advantages compared to the known dosage forms. Particularly, there is a demand of pharmaceutical compositions adapted for parenteral administration of tapentadol.

The stability of the active ingredient in the final product is a primary concern to the formulator. In general, drug substances are less stable in aqueous media than solid dosage forms, and it is important to properly stabilize and preserve liquid aqueous formulations such as solutions, suspensions, and emulsions. Acid-base reactions, acid or base catalysis, oxidation, and reduction can occur in these products. These reactions can arise from drug substance-ingredient interactions, ingredient-ingredient interactions or container-product interactions. For pH sensitive compounds, any of these interactions may alter the pH and may cause precipitation.

Oxidative labile drug substances or vitamins, essential oils, and almost all fats and oils can be oxidized by auto-oxidation. Such reactions can be initiated by heat, light, peroxides, or other labile compounds or heavy metals such as copper or iron.

The effect of trace metals can be minimized by using chelating agents such as EDTA or its sodium or calcium salts. Antioxidants may retard or delay oxidation by rapidly reacting with free radicals as they are formed (quenching). Common antioxidants include propyl, octyl and dodecylesters of gallic acid, butylated hydroxyanisole (BHA), butylated hydroxytoluene (BHT), ascorbic acid, sodium ascorbate, monothioglycerol, potassium or sodium metabisulfite, propionic acid, propyl gallate, sodium bisulfite, sodium sulfite, and the tocopherols or vitamin E.

In addition to stabilization of pharmaceutical preparations against chemical and physical degradation, liquid and semisolid preparations, particularly multiple dosage unit preparations, must usually be protected against microbial contamination. In contrast to solid preparations, aqueous solutions, syrups, emulsions, and suspensions often provide excellent growth media for microorganisms such as molds, yeast, and bacteria (e.g. *Pseudomonas Aeruginosa, E. Coli, Salmonella spp., Staphylococcus aureus, Candida albicans, Aspergillus niger).* Contamination by these microorganisms may occur during manufacturing or when a dose is taken from a multiple dosage unit formulation. Growth of the microorganisms occurs when a sufficient amount of water is present in the formulation.

Ophthalmic and injectable preparations are typically sterilized by autoclaving or filtration. However, many of them require the presence of an antimicrobial preservative to maintain aseptic conditions throughout their stated shelf life, specifically for multiple dosage unit preparations.

When a preservative is required, its selection is based upon several considerations, in particular the site of use whether internal, external or ophthalmic (for further details it can be referred to e.g. Remington, The Science and Practice of Pharmacy, 21st edition, Lippincott Williams & Wilkins, 2005).

Many liquid and semisolid formulations, particularly multiple dosage unit formulations, contain parabens as preservatives, e.g. methyl paraben (methyl-4-hydroxybenzoate) and propyl paraben (propyl-4-hydroxybenzoate).

Because of the number of excipients and additives in pharmaceutical formulations, it is recommended all the ingredients be listed on the container to reduce the risks that confront hypersensitive patients when these products are administered.

Other commercialized pharmaceutical formulations contain sorbic acid or its potassium salt (e.g. Mobilat^{®}) or benzalkonium chloride as preservative. Recently, side effects resulting from mucosal damage caused by benzalkonium chloride and potassium sorbate were reported (cf. C.Y. Ho et al., Am J Rhinol. 2008, 22(2), 125-9). As far as hypersensitivity reactions of preservatives in topical ophthalmic therapies are concerned, quaternary ammoniums (benzalkonium chloride) are commonly associated with irritant toxic reactions whereas the organomercurials (thimerosal) and the alcohols (chlorobutanol) have high associations, respectively, with allergic responses (cf. J. Hong et al., Curr Opin Allergy Clin Immunol. 2009, 9(5), 447-53). Parabens have been implicated in numerous cases of contact sensitivity associated with cutaneous exposure (cf. M.G. Soni et al., Food Chem Toxicol. 2001, 39(6), 513-32) and have been reported to exert a weak estrogenic activity (cf. S. Oishi, Food Chem Toxicol. 2002, 40(12), 1807-13 and M.G. Soni et al., Food Chem Toxicol. 2005, 43(7), 985-015).

Due to these undesired side effects of known preservatives, it is desirable to provide pharmaceutical compositions adapted for parenteral administration of tapentadol that exhibit a sufficient shelf-life in the absence of preservatives or at least in the presence of comparatively low quantities thereof.

It is an object of the invention to provide pharmaceutical formulations of tapentadol that have advantages over the pharmaceutical formulations of the prior art. The pharmaceutical formulations should not have the above preservative based side effects that are typically observed with pharmaceutical formulations containing preservatives such as allergic reactions and should be suitable for parenteral administration of tapentadol.

This object has been achieved by the subject-matter of the patent claims.

It has been surprisingly found that tapentadol as such exhibits preservative properties and thus, when formulating comparatively labile compositions, particularly aqueous liquid or semisolid compositions, preservatives can be completely omitted or at least need to be present in comparatively low amounts in order to achieve the stated shelf-life.

It has been surprisingly found that the antimicrobial activity of tapentadol depends upon the pH value.

Further, it has been surprisingly found that a combination of intracerebroventricular administration and intrathecal administration exhibits a synergistic effect for the treatment of pain.

A first aspect of the invention relates to an aqueous pharmaceutical composition adapted for parenteral administration of tapentadol or a physiologically acceptable salt thereof having a pH value of at least 4.0, preferably of at least 4.5, more preferably of at least 5.0, still more preferably of at least 5.4.

For the purpose of the specification, the term "tapentadol" includes the free base ((1R, 2R)-3-(3-dimethylamino-1-ethyl-2-methyl-propyl)-phenol) as well as any physiologically acceptable salt thereof, particularly the hydrochloride ((1R,2R)-3-(3-dimethylamino-1-ethyl-2-methylpropyl)-phenol hydrochloride). Thus, unless expressly stated otherwise, the term "tapentadol" does not only refer to the free base but also to any physiologically acceptable salt. Further, unless expressly stated otherwise, all amounts, contents and concentrations are equivalents related to tapentadol free base.

Preferably, the content of tapentadol is within the range of from 0.0001 to 20.0 wt.-%, more preferably 0.001 to 15.0 wt.-%, still more preferably 0.005 to 10 wt.-%, yet more preferably 0.01 to 5.0 wt.-%, most preferably 0.05 to 3.0 wt.-% and in particular 0.1 to 2.0 wt.-%, based on the total weight of the composition.

In a preferred embodiment, the content of tapentadol is within the range of from 0.05 to 5 wt.-%, more preferably 0.1 to 4 wt.-%, still more preferably 0.5 to 3.0 wt.-%, yet more preferably 1.0 to 2.5 wt.-%, most preferably 1.25 to 2.25 wt.-% and in particular 1.5 to 2.0 wt.-%, based on the total weight of the composition.

In another preferred embodiment, the content of tapentadol is within the range of from 0.001 to 2.5 wt.-%, more preferably 0.005 to 1.0 wt.-%, still more preferably 0.01 to 0.75 wt.-%, yet more preferably 0.025 to 0.5 wt.-%, most preferably 0.05 to 0.25 wt.-% and in particular 0.075 to 0.15 wt.-%, based on the total weight of the composition. In a preferred embodiment, the content of tapentadol is within the range of from 0.01 to 3.0 wt.-%, more preferably 0.05 to 2.8 wt.-%, still more preferably 0.1 to 2.6 wt.-%, yet more preferably 0.2 to 2.4 wt.-%, most preferably 0.3 to 2.2 wt.-% and in particular 0.4 to 2.0 wt.-%, based on the total weight of the composition.

It has been found that the antimicrobial effect of tapentadol, its preservative effect, is a function of the pH value. Thus, at a given pH value a certain minimum concentration of tapentadol is already sufficient in order to achieve the desired preserving effect, while at another pH value another minimum concentration of tapentadol is necessary in order to achieve the same preserving effect. This minimum concentration for a given pH value can be determined by routine experimentation.

Preferably, the concentration of tapentadol is equal or below 100 mg/mL, more preferably equal or below 75 mg/mL, still more preferably equal or below 50 mg/mL, yet more preferably equal or below 40 mg/mL, and most preferably equal or below 35 mg/mL, and in particular equal or below 30 mg/mL, based on the total volume of the composition.

Preferably, the concentration of tapentadol is within the range of from 0.01 to 100 mg/mL, more preferably within the range of from 0.05 to 75 mg/mL, still more preferably within the range of from 0.1 to 50 mg/mL, yet more preferably within the range of from 0.25 to 30 mg/mL, most preferably within the range of from 0.4 to 25 mg/mL, and in particular within the range of from 0.5 to 20 mg/mL based on the total volume of the composition.

In a preferred embodiment, the concentration of tapentadol is below 25 mg/mL, more preferably below 20 mg/mL, still more preferably at most 19 mg/mL, yet more preferably at most 18 mg/mL, most preferably at most 17 mg/mL, and in particular at most 16 mg/mL based on the total volume of the composition.

In a preferred embodiment, the concentration of tapentadol is within the range of 17.5±6 mg/mL, more preferably 17.5±5 mg/mL, still more preferably 17.5±4 mg/mL, yet more preferably 17.5±3 mg/mL, most preferably 17.5±2 mg/mL, and in particular 17.5±1 mg/mL, based on the total volume of the composition.

In another preferred embodiment, the concentration of tapentadol is within the range of 15±6 mg/mL, more preferably 15±5 mg/mL, still more preferably 15±4 mg/mL, yet more preferably 15±3 mg/mL, most preferably 15±2 mg/mL, and in particular 15±1 mg/mL, based on the total volume of the composition.

In another preferred embodiment, the concentration of tapentadol is within the range of 12.5±6 mg/mL, more preferably 12.5±5 mg/mL, still more preferably 12.5±4 mg/mL, yet more preferably 12.5±3 mg/mL, most preferably 12.5±2 mg/mL, and in particular 12.5±1 mg/mL, based on the total volume of the composition.

In another preferred embodiment, the concentration of tapentadol is within the range of 10±6 mg/mL, more preferably 10±5 mg/mL, still more preferably 10±4 mg/mL, yet more preferably 10±3 mg/mL, most preferably 10±2 mg/mL, and in particular 10±1 mg/mL, based on the total volume of the composition.

In still another preferred embodiment, the concentration of tapentadol is within the range of 5±4 mg/mL, more preferably 5±3 mg/mL, still more preferably 5±2 mg/mL, yet more preferably 5±1.5 mg/mL, most preferably 5±1 mg/mL, and in particular 5±0.5 mg/mL, based on the total volume of the composition.

In yet another preferred embodiment, the concentration of tapentadol is within the range of from 0.01 to 10 mg mg/mL, more preferably 0.025 to 7.5 mg/mL, still more preferably 0.05 to 5.0 mg/mL, yet more preferably 0.1 to 3.0 mg/mL, most preferably 0.25 to 2.0 mg/mL, and in particular 0.5 to 1.5 mg/mL, based on the total volume of the composition.

In a preferred embodiment, the content of tapentadol is within the range of 1.0±0.9 mg/mL, more preferably 1.0±0.8 mg/mL, still more preferably 1.0±0.7 mg/mL, yet more preferably 1.0±0.6 mg/mL, even more preferably 1.0±0.5 mg/mL, most preferably 1.0±0.4 mg/mL, and in particular 1.0±0.3 mg/mL, based on the total weight of the composition.

In a preferred embodiment, the content of tapentadol is within the range of 5.0±4.5 mg/mL, more preferably 5.0±4.0 mg/mL, still more preferably 5.0±3.5 mg/mL, yet more preferably 5.0±3.0 mg/mL, even more preferably 5.0±2.5 mg/mL, most preferably 5.0±2.0 mg/mL, and in particular 5.0±1.5 mg/mL, based on the total weight of the composition.

In a preferred embodiment, the content of tapentadol is within the range of 10±9 mg/mL, more preferably 10±8 mg/mL, still more preferably 10±7 mg/mL, yet more preferably 10±6 mg/mL, even more preferably 10±5 mg/mL, most preferably 10±4 mg/mL, and in particular 10±3 mg/mL, based on the total weight of the composition.

In a preferred embodiment, the content of tapentadol is within the range of 15±14 mg/mL, more preferably 15±12 mg/mL, still more preferably 15±10 mg/mL, yet more preferably 15±8 mg/mL, even more preferably 15±6 mg/mL, most preferably 15±4 mg/mL, and in particular 15±2 mg/mL, based on the total weight of the composition.

The term "pharmaceutical composition" includes any pharmaceutical preparation or formulation that is customized for being administered to a human being or animal. Preferably, the composition is an aqueous solution.

Preferably, the water content of the composition is at least 50 wt.-%, more preferably at least 60 wt.-%, still more preferably at least 70 wt.-%, yet more preferably at least 80 wt.-%, most preferably at least 85 wt.-% and in particular at least 90 wt.-%, based on the total weight of the composition.

In a preferred embodiment, the water content of the composition is at least 90 wt.-%, more preferably at least 92 wt.-%, still more preferably at least 95 wt.-%, yet more preferably at least 96 wt.-%, most preferably at least 98 wt.-% and in particular at least 99 wt.-%, based on the total weight of the composition.

In another preferred embodiment, the water content of the composition is within the range of 90±9 wt.-%, more preferably 90±8 wt.-%, still more preferably 90±7 wt.-%, yet more preferably 90±6 wt.-%, most preferably 90±5 wt.-% and in particular 90±2.5 wt.-%, based on the total weight of the composition.

In still another preferred embodiment, the water content of the composition is within the range of 95±4.5 wt.-%, more preferably 95±4 wt.-%, still more preferably 95±3.5 wt.-%, yet more preferably 95±3 wt.-%, most preferably 95±2 wt.-% and in particular 95±1 wt.-%, based on the total weight of the composition.

In yet another preferred embodiment, the water content of the composition is within the range of 98±1.9 wt.-%, more preferably 98±1.5 wt.-%, still more preferably 98±1.25 wt.-%, yet more preferably 98±1.0 wt.-%, most preferably 98±0.75 wt.-% and in particular 98±0.5 wt.-%, based on the total weight of the composition.

Besides water, the composition according to the invention may contain further solvents.

Further suitable solvents include all types of physiologically acceptable hydrophilic solvents, preferably selected from the group consisting of ethanol, glycerol, propylene glycol, 1,3-butanediol and macrogol 300.

In a preferred embodiment, the composition according to the invention is adapted for local administration. In this regard, local administration includes every administration of the composition to a site which is identical to the site of disorder and/or at least is located nearby. In particular, the local administration has the purpose of delivering tapentadol directly to the desired site of action, thereby avoiding systemic side-effects.

Preferably, the systemic concentration of tapentadol is kept at a sub-therapeutic concentration; i. e. during the treatment, the systemic concentration of tapentadol never reaches the level that is required for exhibiting a therapeutic effect when the drug is only administered systemically.

In another preferred embodiment, the composition according to the invention is adapted for systemic administration. In this embodiment the administration of the composition preferably has the purpose of inducing a systemic action of tapentadol.

The composition according to the invention is adapted for parenteral administration, preferably by infusion or injection.

In order to satisfy high quality requirements for infusion and injection solutions, respectively, the composition has to exhibit a physiologically acceptable osmolarity and a physiologically acceptable pH.

Isotonic sodium chloride solution (saline), for instance, contains 0.9 wt.-% of sodium chloride and exhibits an osmolarity of 0.308 osmol/L, which is close to the osmolarity of blood.

Preferably, the composition has an osmolarity of at least 0.22 osmol/L, more preferably of at least 0.23 osmol/L, still more preferably of at least 0.24 osmol/L, yet more preferably of at least 0.25 osmol/L, most preferably of at least 0.26 osmol/L, and in particular of at least 0.27 osmol/L.

In a preferred embodiment, the composition has an osmolarity of at most 0.36 osmol/L, more preferably of at most 0.34 osmol/L, still more preferably of at most 0.32 osmol/L, yet more preferably of at most 0.31 osmol/L, most preferably of at most 0.30 osmol/L and in particular of at most 0.29 osmol/L.

In another preferred embodiment, the composition has an osmolarity of 0.28±0.08 osmol/L, more preferably of 0.28±0.06 osmol/L, still more preferably of 0.28±0.04 osmol/L, yet more preferably of 0.28±0.03 osmol/L, most preferably of 0.28±0.02 osmol/L, and in particular of 0.28±0.01 osmol/L.

The osmolarity of the composition depends on the content of tapentadol and optionally the buffer and is preferably adjusted during the manufacture of the composition by the addition of an appropriate amount of sodium chloride. Other isotonizing agents such as mannitol or sorbitol can also be added alternatively or additionally.

Preferably, the composition according to the invention further contains sodium chloride.

Preferably, the content of the sodium chloride is at most 2.0 wt.-%, more preferably at most 1.75 wt.-%, still more preferably at most 1.5 wt.-%, yet more preferably at most 1.3 wt.-%, most preferably at most 1.1 wt.-%, and in particular at most 1.0 wt.-%, based on the total weight of the composition.

Preferably, the sodium chloride has a concentration within the range of from 0.5 mg/mL to 25 mg/mL, more preferably from 1.0 mg/mL to 20 mg/mL, still more preferably from 2.0 mg/mL to 15 mg/mL, most preferably from 2.5 mg/mL to 12 mg/mL, and in particular from 3 mg/mL to 10 mg/mL, based on the total volume of the composition.

In a preferred embodiment, the sodium chloride has a concentration within the range of 9.0±8.0 mg/mL, more preferably 9.0±5.0 mg/mL, still more preferably 9.0±3.0 mg/mL, yet more preferably 9.0±2.0 mg/mL, most preferably 9.0±1.0 mg/mL, and in particular 9.0±0.5 mg/mL, based on the total volume of the composition.

In another preferred embodiment, the sodium chloride has a concentration within the range of 8.0±7.0 mg/mL, more preferably 8.0±5.0 mg/mL, still more preferably 8.0±3.0 mg/mL, yet more preferably 8.0±2.0 mg/mL, most preferably 8.0±1.0 mg/mL, and in particular 8.0±0.5 mg/mL, based on the total volume of the composition.

In still another preferred embodiment, the sodium chloride has a concentration within the range of 6.0±5.0 mg/mL, more preferably 6.0±4.0 mg/mL, still more preferably 6.0±3.0 mg/mL, yet more preferably 6.0±2.0 mg/mL, most preferably 6.0±1.0 mg/mL, and in particular 6.0±0.5 mg/mL, based on the total volume of the composition.

In yet another preferred embodiment, the sodium chloride has a concentration within the range of 5.0±4.5 mg/mL, more preferably 5.0±4.0 mg/mL, still more preferably 5.0±3.5 mg/mL, yet more preferably 5.0±2.0 mg/mL, most preferably 5.0±1.0 mg/mL, and in particular 5.0±0.5 mg/mL, based on the total volume of the composition.

In a preferred embodiment, the composition has a pH value of at least 4.00, of at least 4.25, of at least 4.50, of at least 4.75, of at least 5.00, of at least 5.25, or of at least 5.50; more preferably at least 5.75, still more preferably at least 6.00, yet more preferably at least 6.25, even more preferably at least 6.50, most preferably at least 6.75, and in particular at least 7.00.

In another preferred embodiment, the composition has a pH value of at most 7.00, more preferably of at most 6.75, still more preferably of at most 6.50, yet more preferably of at most 6.25, and even more preferably of at most 6.00.

Preferably, the composition has a pH value within the range of from 5.4 to 6.5, more preferably 5.5 to 6.3, still more preferably 5.4 to 6.0.

In a preferred embodiment, the composition has a pH value within the range of 5.0±1.0, more preferably 5.0±0.9, still more preferably 5.0±0.8, yet more preferably 5.0±0.7, even more preferably 5.0±0.6 or 5.0±0.5, most preferably 5.0±0.4 or 5.0±0.3, and in particular 5.0±0.2 or 5.0±0.1.

In a preferred embodiment, the composition has a pH value within the range of 5.5±1.0, more preferably 5.5±0.9, still more preferably 5.5±0.8, yet more preferably 5.5±0.7, even more preferably 5.5±0.6 or 5.5±0.5, most preferably 5.5±0.4 or 5.5±0.3, and in particular 5.5±0.2 or 5.5±0.1.

In a preferred embodiment, the composition has a pH value within the range of 5.7±0.3, more preferably 5.7±0.25, still more preferably 5.7±0.2, most preferably 5.7±0.15, and in particular 5.7±0.1.

In a preferred embodiment, the composition has a pH value within the range of 6.0±0.6, more preferably 6.0±0.5, still more preferably 6.0±0.4, even more preferably 6.0±0.3, most preferably 6.0±0.2, and in particular 6.0±0.1.

In a preferred embodiment, the composition has a pH value within the range of 6.5±1.0, more preferably 6.5±0.9, still more preferably 6.5±0.8, yet more preferably 6.5±0.7, even more preferably 6.5±0.6 or 6.5±0.5, most preferably 6.5±0.4 or 6.5±0.3, and in particular 6.5±0.2 or 6.5±0.1.

In a preferred embodiment, the composition has a pH value within the range of 7.0±1.4 or 7.0±1.3, more preferably 7.0±1.2 or 7.0±1.1, still more preferably 7.0±1.0 or 7.0±0.9, yet more preferably 7.0±0.8 or 7.0±0.7, even more preferably 7.0±0.6 or 7.0±0.5, most preferably 7.0±0.4 or 7.0±0.3, and in particular 7.0±0.2 or 7.0±0.1.

In a preferred embodiment, the composition has a pH value within the range of 7.5±1.4 or 7.5±1.3, more preferably 7.5±1.2 or 7.5±1.1, still more preferably 7.5±1.0 or 7.5±0.9, yet more preferably 7.5±0.8 or 7.5±0.7, even more preferably 7.5±0.6 or 7.5±0.5, most preferably 7.5±0.4 or 7.5±0.3, and in particular 7.5±0.2 or 7.5±0.1.

In a preferred embodiment, the composition has a pH value within the range of 8.0±1.4 or 8.0±1.3, more preferably 8.0±1.2 or 8.0±1.1, still more preferably 8.0±1.0 or 8.0±0.9, yet more preferably 8.0±0.8 or 8.0±0.7, even more preferably 8.0±0.6 or 8.0±0.5, most preferably 8.0±0.4 or 8.0±0.3, and in particular 8.0±0.2 or 8.0±0.1.

In a preferred embodiment, the composition has a pH value within the range of 8.5±1.4 or 8.5±1.3, more preferably 8.5±1.2 or 8.5±1.1, still more preferably 8.5±1.0 or 8.5±0.9, yet more preferably 8.5±0.8 or 8.5±0.7, even more preferably 8.5±0.6 or 8.5±0.5, most preferably 8.5±0.4 or 8.5±0.3, and in particular 8.5±0.2 or 8.5±0.1.

It has been surprisingly found that tapentadol exhibits a pH-dependent antimicrobial effect. Thus, the pH value of the composition according to the invention is preferably adjusted to a value within the physiologically acceptable range where the antimicrobial effect of tapentadol is maximized.

Preferably, the composition according to the invention is buffered, i.e. contains one or more buffers and buffer systems (i.e. conjugate acid-base-pairs), respectively. Preferred buffer systems are derived from the following acids: organic acids such as acetic acid, propionic acid, maleic acid, fumaric acid, lactic acid, malonic acid, malic acid, mandelic acid, citric acid, tartric acid, succinic acid; or inorganic acids such as phosphoric acid. When the buffer systems are derived from any of the above acids, the buffer system constitutes of said acid and its conjugate base. Buffer systems derived from acetic acid, citric acid, lactic acid, succinic acid or phosphoric acid are particularly preferred.

A skilled person is fully aware that multiprotonic acids can form more than a single buffer system. For example, citric acid is a triprotonic acid so that it forms the conjugate acid-base pairs citric acid - dihydrogencitrate, dihydrogencitrate - hydrogencitrate and hydrogencitrate - citrate. In other words, any of citric acid, dihydrogencitrate and hydrogencitrate can be the acid of a buffer system with the conjugate base. For the purpose of the specification, the expression "buffer and buffer system, respectively" preferably refers to the quantity of both, the acid and its conjugate base. Further, a skilled person is fully aware that a buffer system, e.g. the conjugate system citric acid/sodium dihydrogencitrate can be established either by adding citric acid and an appropriate amount of sodium hydroxide, or sodium citrate and an appropriate amount of hydrochloric acid, or citric acid and sodium dihydrogencitrate as such.

Accordingly, in case that the composition contains an appropriate amount of tapentadol in form of its hydrochloride, a buffer system can be established by adding sodium citrate or its dihydrate.

Preferably, the concentration of the buffer and buffer system, respectively, preferably sodium citrate or its dihydrate or sodium acetate, is adjusted to provide a sufficient buffer capacity.

In a preferred embodiment, the content of the buffer and buffer system, respectively, preferably sodium citrate or its dihydrate or sodium acetate, is within the range of from 0.0001 to 5.0 wt.-%, more preferably 0.0002 to 2.5 wt.-%, still more preferably 0.0005 to 1.0 wt.-%, yet more preferably 0.001 to 0.5 wt.-%, most preferably 0.005 to 0.25 wt.-% and in particular 0.01 to 0.1 wt.-%, based on the total weight of the composition.

In another preferred embodiment, the content of the buffer and buffer system, respectively, preferably sodium citrate or its dihydrate or sodium acetate, is within the range of from 0.0001 to 5.0 wt.-%, more preferably 0.0002 to 4.0 wt.-%, still more preferably 0.0005 to 3.0 wt.-%, yet more preferably 0.001 to 2.0 wt.-%, most preferably 0.005 to 1.0 wt.-% and in particular 0.05 to 0.55 wt.-%, based on the total weight of the composition.

In a preferred embodiment, the buffer and buffer system, respectively, preferably sodium citrate or its dihydrate or sodium acetate, has a concentration within the range of 1.0±0.6 mg/mL, more preferably 1.0±0.5 mg/mL, still more preferably 1.0±0.4 mg/mL, yet more preferably 1.0±0.3 mg/mL, most preferably 1.0±0.2 mg/mL, and in particular 1.0±0.1 mg/mL, based on the total volume of the composition.

In another preferred embodiment, the buffer and buffer system, respectively, preferably sodium citrate or its dihydrate or sodium acetate, has a concentration within the range of 0.8±0.6 mg/mL, more preferably 0.8±0.5 mg/mL, still more preferably 0.8±0.4 mg/mL, yet more preferably 0.8±0.3 mg/mL, most preferably 0.8±0.2 mg/mL, and in particular 0.8±0.1 mg/mL, based on the total volume of the composition.

In another preferred embodiment, the buffer and buffer system, respectively, preferably sodium citrate or its dihydrate or sodium acetate, has a concentration within the range of 0.6±0.55 mg/mL, more preferably 0.6±0.5 mg/mL, still more preferably 0.6±0.4 mg/mL, yet more preferably 0.6±0.3 mg/mL, most preferably 0.6±0.2 mg/mL, and in particular 0.6±0.1 mg/mL, based on the total volume of the composition.

In another preferred embodiment, the buffer and buffer system, respectively, preferably sodium citrate or its dihydrate or sodium acetate, has a concentration within the range of 0.5±0.45 mg/mL, more preferably 0.5±0.4 mg/mL, still more preferably 0.5±0.35 mg/mL, yet more preferably 0.5±0.3 mg/mL, most preferably 0.5±0.25 mg/mL, and in particular 0.5±0.1 mg/mL, based on the total volume of the composition.

In another preferred embodiment, the buffer and buffer system, respectively, preferably sodium citrate or its dihydrate or sodium acetate, has a concentration within the range of 0.4±0.35 mg/mL, more preferably 0.4±0.3 mg/mL, still more preferably 0.4±0.25 mg/mL, yet more preferably 0.4±0.2 mg/mL, most preferably 0.4±0.15 mg/mL, and in particular 0.4±0.1 mg/mL, based on the total volume of the composition.

In still another preferred embodiment, the buffer and buffer system, respectively, preferably sodium citrate or its dihydrate or sodium acetate, has a concentration within the range of 0.3±0.25 mg/mL, more preferably 0.3±0.2 mg/mL, still more preferably 0.3±0.15 mg/mL, most preferably 0.3±0.1 mg/mL, and in particular 0.3±0.05 mg/mL, based on the total volume of the composition.

In yet another preferred embodiment, the buffer and buffer system, respectively, preferably sodium citrate or its dihydrate or sodium acetate, has a concentration within the range of 0.15±0.14 mg/mL, more preferably 0.15±0.13 mg/mL, still more preferably 0.15±0.12 mg/mL, most preferably 0.15±0.10 mg/mL, and in particular 0.15±0.05 mg/mL, based on the total volume of the composition.

Preferably, the composition does not contain any preservative. For the purpose of the specification, a "preservative" preferably refers to any substance that is usually added to pharmaceutical compositions in order to preserve them against microbial degradation or microbial growth. In this regard, microbial growth typically plays an essential role, i.e. the preservative serves the main purpose of avoiding microbial contamination. As a side aspect, it may also be desirable to avoid any effect of the microbes on the active ingredients and excipients, respectively, i.e. to avoid microbial degradation.

Representative examples of preservatives include benzalkonium chloride, benzethonium chloride, benzoic acid, sodium benzoate, benzyl alcohol, bronopol, cetrimide, cetylpyridinium chloride, chlorhexidine, chlorbutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, sodium propionate, thimerosal, methyl paraben, ethyl paraben, propyl paraben, butyl paraben, isobutyl paraben, benzyl paraben, sorbic acid, and potassium sorbate.

It has been surprisingly found that tapentadol as such exhibits preservative properties and that the antimicrobial activity of tapentadol depends upon the pH value of the composition.

The complete absence of preservatives in the composition is preferred when the content of tapentadol is sufficiently high and the composition has an appropriate pH value so that due to its preservative property the desired shelf life or in use stability can be achieved by the presence of the drug itself. As already mentioned above, the preservative property of tapentadol is a function of the pH value and thus, at one pH value the addition of another preservative might be necessary, whereas at another pH value it can be completely omitted. Preferably, under these circumstances the concentration of tapentadol is at least 1.0 mg/mL or at least 5.0 mg/mL, more preferably at least 10 mg/mL, at least 12 mg/mL, or at least 14 mg/mL, based on the total volume of the composition.

For the purpose of the specification, it is preferably distinguished between shelf life and in-use stability. Shelf life preferably refers to the storage stability of a closed container of the pharmaceutical composition. In-use stability preferably refers to the storage container that contains a multiple dosage unit preparation which has been utilized for the first time. Typically, the shelf-life of a multiple dosage unit preparation is much longer than its in-use stability.

In another preferred embodiment, the composition additionally contains a preservative, which is preferably selected from the group consisting of benzalkonium chloride, benzethonium chloride, benzoic acid, sodium benzoate, benzyl alcohol, bronopol, cetrimide, cetylpyridinium chloride, chlorhexidine, chlorbutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, sodium propionate, thimerosal, methyl paraben, ethyl paraben, propyl paraben, butyl paraben, isobutyl paraben, benzyl paraben, sorbic acid, and potassium sorbate.

It has been surprisingly found that aqueous tapentadol compositions containing sodium benzoate show less total degradation products compared with aqueous tapentadol compositions containing parabens.

Thus, sodium benzoate is a particularly preferred preservative according to the invention.

Preferably, the content of the preservative, preferably benzoic acid or its sodium salt, is at most 5.0 wt.-%, more preferably at most 4.0 wt.-%, still more preferably at most 3.0 wt.-%, yet more preferably at most 2.0 wt.-%, most preferably at most 1.0 wt.-% and in particular at most 0.5 wt.-%, based on the total weight of the composition. The content may depend upon the pH value of the composition.

In a preferred embodiment, the content of the preservative is at most 90%, more preferably at most 80%, still more preferably at most 70%, yet more preferably at most 60%, most preferably at most 50% and in particular at most 40% of the content that would be needed according to Ph. Eur. in order to sufficiently preserve the pharmaceutical composition in the absence of tapentadol, either concerning its shelf-life or, in case of multiple dosage unit preparations, optionally concerning its in-use stability. The criteria for sufficient preservation according to Ph. Eur. are met, if (a) the concentrations of viable bacteria are reduced to not more than 0.1% of the initial concentrations by the seventh day; and (b) the concentration of each test microorganism remains at or below these designated levels during the remainder of the 28-day test period. These criteria are more specifically defined in the experimental section.

Preferably, the composition according to the invention exhibits an antimicrobial robustness that complies with the requirements of the Ph. Eur., preferably in its version for 2010. Preferably, antimicrobial robustness is achieved against *S*. *aureus, Ps. Aeruginosa, S*. *spp., C. albicans,* and/or *A. niger,* preferably satisfying the requirement of log reduction of 1, preferably 3 after 7 and no increase after 28 days. In a particularly preferred embodiment, antimicrobial robustness is achieved against bacteria satisfying the requirement of log reduction of 3 after 14 days and against molds and yeast of log reduction of 1 after 14 days.

Preferably, the composition according to the invention exhibits a shelf-life under accelerated storage conditions of at least 1 month, more preferably at least 2 months, still more preferably at least 3 months, yet more preferably at least 4 months, most preferably at least 5 months and in particular at least 6 months. Preferably, the shelf life is determined according to Ph. Eur., particularly as described in the experimental section. Accelerated storage conditions preferably mean 40±2 °C/75% RH.

Preferably, the composition according to the invention exhibits a shelf-life under ambient conditions of at least 6 month, more preferably at least 12 months, still more preferably at least 15 months, yet more preferably at least 18 months, most preferably at least 21 months and in particular at least 24 months.

Preferably, the composition according to the invention is a multiple dosage unit preparation that exhibits an in-use stability under ambient conditions of at least 1 week, more preferably at least 2 weeks, still more preferably at least 3 weeks, yet more preferably at least 4 weeks, most preferably at least 5 weeks and in particular at least 6 weeks.

Particularly preferred embodiments E¹ to E⁸ concerning the composition according to the invention are summarized in the table here below:

| | E¹ | E² | E³ | E⁴ |
|---|---|---|---|---|
| tapentadol | ≤ 100 mg/mL | ≤ 50 mg/mL | ≤ 30 mg/mL | ≤ 30 mg/mL |
| buffer | optional | 0.0001 - 5 wt.% | 0.0005 - 1 wt.% | 0.001 - 0.5 wt.% |
| sodium chloride | 0.5 - 25 mg/mL | 0.5 - 25 mg/mL | 1 - 20 mg/mL | 1 - 20 mg/mL |
| water | ≥ 90 wt.% | ≥ 90 wt.% | ≥ 95 wt.% | ≥ 95 wt.% |

| | E⁵ | E⁶ | E⁷ | E⁸ |
|---|---|---|---|---|
| tapentadol | ≤ 20 mg/mL | 0.5 - 20 mg/mL | 0.5 - 5.0 mg/mL | 15±6 mg/mL |
| buffer | 0.001-0.5 wt.% | 0.5±0.4 mg/mL sodium citrate or its dihydrate | 0.15±0.1 mg/mL sodium citrate or its dihydrate | 0.5±0.2 mg/mL sodium citrate or its dihydrate |
| sodium chloride | 3 - 10 mg/mL | 3 - 10 mg/ml | 9±2 mg/mL | 5±2 mg/mL |
| water | ≥ 95 wt.% | ≥ 98 wt.% | ≥ 98 wt.% | ≥ 98 wt.% |

In a preferred embodiment, the composition according to the invention is adapted for administration in combination with an anesthetic.

Thus, a further aspect of the invention relates to a combination comprising as components (a) tapentadol, and (b) an anesthetic, preferably lidocaine, irrespective of whether the combination is an aqueous pharmaceutical composition adapted for parenteral administration and/or whether it has a pH value of at least 5.4. Preferably, however, the combination is an aqueous pharmaceutical composition adapted for parenteral administration and/or has a pH value of at least 5.4.

It has been found that a combination comprising (a) tapentadol, and (b) lidocaine or a derivative thereof exhibits an analgesic effect. If these components are present in the combination in such a weight ratio that a synergistic effect is observed after administration to the patients, the overall administered dose may be lowered, so that fewer undesired side-effects will occur.

Both components (a) and (b) as part of the inventive combination may be administered in their usual daily dosage.

In another embodiment of the present invention the inventive combination may contain components (a) and (b) essentially in an equieffective ratio.

In yet a further embodiment of the inventive combination components (a) and (b) are present in such a weight ratio that the resulting composition will exert a synergistic effect upon administration to a patient. Suitable weight ratios can be determined by methods well known to those skilled in the art, e.g. via the Randall-Selitto test.

Both components (a) and (b) may also be present in the inventive combination in ratios deviating from the equieffective ratio. For, example, each of the components could be present in a range from 1/5 of the equieffective amount to 5 times the equieffective amount, preferably 1/4 to 4, more preferably 1/3 to 3, yet more preferably 1/2 to 2 of the equieffective amount.

In another embodiment, the components (a) and (b) can be administered in a specific dosage regimen to treat pain, for example, diabetic neuropathic pain, cancer pain, perioperative and/or post-operative pain. Components (a) and (b) may be administered simultaneously or sequentially to one another, in each case via the same or different administration pathways. Another aspect of the present invention is therefore a method of treating pain, e.g. diabetic neuropathic pain, cancer pain, perioperative and/or post-operative pain, characterized in that components (a) and (b) are administered simultaneously or sequentially to a mammal, wherein component (a) may be administered before or after component (b) and wherein components (a) or (b) are administered to the mammal either via the same or a different pathway of administration. Suitable pathways of administrations include but are not limited to oral, intravenous, intraperitoneal, transdermal, intrathekal, intramuscular, intranasal, transmucosal, subcutaneous, or rectal administration.

The inventive combinations are toxicologically safe and are therefore suitable for the treatment of mammals, particularly humans including infants, children and grown-ups.

Preferably, the anesthetic is selected from the group consisting of diethyl ether, vinyl ether, halothane, chloroform, methoxyflurane, enflurane, trichioroethylene, isoflurane, desfiurane, sevoflurane, methohexital, hexobarbital, thiopental, narcobarbital, fentanyl, alfentanil, sufentanil, phenoperidine, anileridine, remifentanil, droperidol, ketamine, propanidid, alfaxalone, etomidate, propofol, hydroxybutyric acid, nitrous oxide, esketamine, metabutethamine, procaine, tetracaine, chioroprocaine, benzocaine, bupivacaine, lidocaine, mepivacaine, prilocaine, butanilicaine, cinchocaine, etidocaine, articaine, ropivacaine, levobupivacaine, cocaine, ethyl chloride, dyclonine, phenol, and capsaicin.

In a preferred embodiment, the anesthetic is a local anesthetic selected from the group consisting of lidocaine, mepivacaine, prilocaine, articaine, bupivacaine, ropivacaine, etidocaine, dyclonine, procaine, benzocaine, 2-chloroprocaine, tetracaine, and formocain. Especially preferred is lidocaine.

Preferably, the dosage of tapentadol relative to the dosage of the anesthetic is within the range of from 100:1 to 1:100, more preferably 80:1 to 1:80, still more preferably 60:1 to 1:60, yet more preferably 40:1 to 1:40, most preferably 20:1 to 1:20, and in particular 10:1 to 1:10.

In a preferred embodiment, the dosage of tapentadol relative to the dosage of the anesthetic is within the range of 100±80:1, more preferably 100±60:1, still more preferably 100±40:1, yet more preferably 100±30:1, most preferably 100±20:1, and in particular 100±10:1.

In another preferred embodiment, the dosage of tapentadol relative to the dosage of the anesthetic is within the range of 75±60:1, more preferably 75±50:1, still more preferably 75±40:1, yet more preferably 75±30:1, most preferably 75±20:1, and in particular 75±10:1.

In still another preferred embodiment, the dosage of tapentadol relative to the dosage of the anesthetic is within the range of 50±40:1, more preferably 50±30:1, still more preferably 50±25:1, yet more preferably 50±20:1, most preferably 50±15:1, and in particular 50±10:1.

In yet another preferred embodiment, the dosage of tapentadol relative to the dosage of the anesthetic is within the range of 25±20:1, more preferably 25±15:1, still more preferably 25±12.5:1, yet more preferably 25±10:1, most preferably 25±7.5:1, and in particular 25±5:1.

In a preferred embodiment, the dosage of the anesthetic relative to the dosage of tapentadol is within the range of 100±80:1, more preferably 100±60:1, still more preferably 100±40:1, yet more preferably 100±30:1, most preferably 100±20:1, and in particular 100±10:1.

In another preferred embodiment, the dosage of the anesthetic relative to the dosage of tapentadol is within the range of 75±60:1, more preferably 75±50:1, still more preferably 75±40:1, yet more preferably 75±30:1, most preferably 75±20:1, and in particular 75±10:1.

In still another preferred embodiment, the dosage of the anesthetic relative to the dosage of tapentadol is within the range of 50±40:1, more preferably 50±30:1, still more preferably 50±25:1, yet more preferably 50±20:1, most preferably 50±15:1, and in particular 50±10:1.

In yet another preferred embodiment, the dosage of the anesthetic relative to the dosage of tapentadol is within the range of 25±20:1, more preferably 25±15:1, still more preferably 25±12.5:1, yet more preferably 25±10:1, most preferably 25±7.5:1, and in particular 25±5:1.

In a preferred embodiment, the dosage of tapentadol relative to the dosage of the anesthetic is within the range of from 25:1 to 1:25, more preferably 10:1 to 1:20, still more preferably 5:1 to 1:15, yet more preferably 1:1 to 1:10, most preferably 1:2 to 1:8, and in particular 1:3 to 1:6.

In a preferred embodiment, the anesthetic and the tapentadol are contained in a single composition (for the purpose of the specification also referred to as "combined composition").

In another preferred embodiment, the anesthetic and the tapentadol are contained in two separate pharmaceutical compositions. For the purpose of the specification these separate pharmaceutical compositions are also referred to as "first composition" (contains component (a), i.e. the tapentadol) and "second composition" (contains component (b), i. e. the anesthetic)}.

Preferably, the combined composition and the first composition, respectively, contain the aqueous pharmaceutical composition according to the invention, i. e. all preferred embodiments that are described above in connection with the composition according to the invention also apply to the combined composition as well as to the first composition, respectively.

Unless expressly stated otherwise, the general terms "composition" and "pharmaceutical composition", respectively, as mentioned hereafter, refer to all embodiments referring to compositions containing the tapentadol, i.e. the aqueous pharmaceutical composition, the combined composition as well as the first composition, respectively.

In a preferred embodiment, the anesthetic is contained in a second, separate composition, which is preferably adapted for parenteral administration.

The first composition according to the invention may be administered prior to, simultaneously with and/or subsequently to the second composition according to the invention. It is also possible to divide the first and the second composition in subunits and to administer some subunits prior to, simultaneously with and/or subsequently to other subunits. For example, the first composition may be divided into two subunits and the first subunit is administered in the beginning. Thereafter, the second composition is administered. The second subunit of the first composition may then either be administered simultaneously with or subsequently to the second composition.

The present invention also encompasses administration regimens that combine the administration of a combined composition according to the invention prior to, simultaneously with and/or subsequently to administration of a first and/or second pharmaceutical composition according to the invention, respectively.

Independent of the order of administration, the length of the period between administration of both, i.e. first and second pharmaceutical compositions is preferably at most 60 minutes, more preferably of at most 45 minutes, still more preferably of at most 30 minutes, yet more preferably of at most 15 minutes, most preferably of at most 10 minutes, and in particular of at most 5 minutes.

In a preferred embodiment, the first composition comprising the tapentadol and the second, separate composition comprising the anesthetic are contained in a kit.

Preferably, the first composition and the second composition are both injection solutions or infusion solutions.

A further aspect of the invention relates to a pharmaceutical dosage form comprising the pharmaceutical composition according to the invention, preferably the aqueous pharmaceutical composition, the combined composition or the first composition according to the invention. All preferred embodiments that are described above in connection with the composition according to the invention also apply to the dosage form according to the invention.

Preferably, the dosage form is selected from the group consisting of injection solutions, injection suspensions, infusion solutions, infusion suspensions, and depot formulations, such as depot injection solutions, depot injection suspensions, implants and infusion pumps.

Compared to oral dosage forms, parenteral dosage forms have several advantages, especially when the patient is young or has problems to swallow. They can be exactly dosed, e.g. according to the body weight of the patients, which can be particularly important in pediatric patients. Further, they can be administered by infusion continually over an extended period of time (e. g. 24 h), e. g. by means of an infusion pump.

In a preferred embodiment, the dosage form is a multiple dosage unit form, i.e. customized for more than a single administration, preferably by injection.

For the purpose of the specification "multiple dosed" preferably means that the dosage form encompasses more than a single dosage unit.

For example, when the dosage form is a multiple dosed injection solution, its overall volume is more than the volume that is to be typically administered at once. Instead, the multiple dosed injection solution is customized for being divided into a multitude of dosage units that are to be administered over a treatment interval typically encompassing several days. The individual dosage units may preferably be separated from the multiple dosage unit form by means of a syringe. A typical example for a multiple dosage form according to the invention is an optionally sterilized glass container sealed with a septum. Said glass container contains a volume of the pharmaceutical combination well exceeding the individual volume of an individual dosage unit that is intended for at once administration to the patient. For example, when the multiple dosed dosage form that is contained in a storage container has a total volume of 250 mL and the prescribed dosage unit is 25 mL once daily, at day 1 of the treatment interval the patient takes 25 mL so that 225 mL remain in the storage container; at day 2 of the treatment interval the patient takes another 25 mL so that 200 mL remain in the storage container; and so on, until at day 10 the entire amount is taken by the patient.

Preferably, the multiple dosage unit form contains at least 2, more preferably at least 3, even more preferably at least 5, yet more preferably at least 10, most preferably at least 12, and in particular at least 15 individual dosage units.

Preferably, the individual dosage units have a volume of 0.25 mL to 3.0 mL, more preferably of 0.5 mL to 2.75 mL, still more preferably of 0.75 mL to 2.5 mL, and most preferably of 1.0 mL to 2.0 mL.

In a preferred embodiment, the individual dosage units have a volume of 1.0±0.9 mL, more preferably of 1.0±0.75 mL, still more preferably 1.0±0.5 mL, yet more preferably of 1.0±0.4 mL, even more preferably of 1.0±0.2 mL, most preferably of 1.0±0.15 mL, and in particular of 1.0±0.1 mL.

In a preferred embodiment, the individual dosage units have a volume of 2.0±0.9 mL, more preferably of 2.0±0.75 mL, still more preferably 2.0±0.5 mL, yet more preferably of 2.0±0.4 mL, even more preferably of 2.0±0.2 mL, most preferably of 2.0±0.15 mL, and in particular of 2.0±0.1 mL.

The individual dosage units may be administered once, twice, thrice, four times, five times, six times or even more frequently, optionally in regular time intervals.

The multiple dosage unit form may also be customized for a continual administration, preferably by infusion. Preferably, the dosage form is adapted for a continual administration for at least 30 minutes or 45 minutes, more preferably for at least 1 h or 2 h, still more preferably for at least 3 h or 4 h, yet more preferably for at least 6 h or 8 h, most preferably for at least 10 h, and in particular for at least 12 h.

In still another preferred embodiment, the dosage form provides a single, individual dosage unit, which is administered as such (single dosage unit form).

The tapentadol is administered in a therapeutically effective amount. The amount that constitutes a therapeutically effective amount varies according to the condition being treated, the severity of said condition and the patient being treated.

The amount of the tapentadol that is contained in the individual dosage unit is preferably within the range of from 10 mg to 250 mg, more preferably within the range of from 15 mg to 200 mg, still more preferably within the range of from 20 mg to 150 mg, yet more preferably within the range of from 30 mg to 130 mg, and most preferably within the range of from 40 mg to 115 mg, and in particular within the range of from 50 mg to 100 mg.

Preferably, the daily dose of the tapentadol is at most 250 mg, more preferably at most 225 mg, yet more preferably at most 200 mg, still more preferably at most 175 mg, and in particular at most 150 mg.

Preferably, the daily dose of the tapentadol is at least 15 mg, more preferably at least 20 mg, yet more preferably at least 25 mg, still more preferably at least 30 mg, most preferably at least 30 mg, and in particular at least 40 mg.

In a preferred embodiment, the dosage form is an infusion solution or infusion suspension.

In another preferred embodiment, the dosage form is an injection solution or injection suspension, which preferably is a single dosage unit form or multiple dosage unit form. Multiple dosage unit injection solutions are preferably contained in an injection vial, whereas single dosage unit forms are preferably contained in a single-use syringe.

In still another preferred embodiment, the dosage form is an implantable device, such as an implantable infusion pump.

In a preferred embodiment, the dosage form according to the invention is a depot formulation (retard formulation).

Preferably, the depot formulation is an infusion solution or infusion suspension, preferably customized for an intramuscular or subcutaneous administration.

Preferably, the depot formulation further contains viscosity-enhancing excipients, such as methylcellulose, gelatine, and polyvidon (polyvinylpyrrolidon) preferably having a molecular weight of at most 40,000 g/mol. By choosing the appropriate type and the appropriate amount of the viscosity-enhancing excipient, the depot effect of the depot formulation may be influenced.

Preferably, the depot formulation is capable of releasing the drug over time period of at least 12 h or 14 h, more preferably at least 16 h or 18 h, still more preferably at least 20 h, yet more preferably at least 24 h, most preferably at least 36 h, and in particular at least 48 h.

The depot formulation is preferably administered for use in the treatment of post-surgical pain.

In a preferred embodiment, the dosage form according to the invention is adapted for administration to pediatric patients. For the purpose of the specification, pediatric patients preferably encompass premature infants, infants, children, and adolescents.

Preferably, the upper age limit of the pediatric patients is 1, 2, 3, 4, 5, 6, 8, 10, 12, 14, 16 or 17.

Preferably, the lower body weight limit of the pediatric patients is 30 kg or 25 kg, more preferably 20 kg or 15 kg, still more preferably 10 kg or 7.5 kg, yet more preferably 5 kg or 3 kg, most preferably 2 kg or 1 kg, and in particular 500 g.

In a preferred embodiment, the dosage form is adapted for administration to children having a body weight of 10 to 15 kg, 16 to 20 kg, 21 to 25 kg, 26 to 30 kg, 31 to 35 kg, 36 to 40 kg and/or 41 to 45 kg.

In another preferred embodiment, the dosage form is adapted for administration to infants or children having a body weight of below 0.5 kg, 0.6 kg to 0.9 kg, 1.0 kg to 1.9 kg, 2.0 kg to 2.9 kg, 3.0 kg to 3.9 kg, 4.0 kg to 4.9 kg, 5.0 kg to 5.9 kg, 6.0 kg to 8.0 kg and/or 8.1 kg to 9.9 kg.

In this regard, the surprising preservative properties of tapentadol are even more beneficial, as the drug approval authorities have set stricter standards as to the presence of preservative in medicaments for pediatric patients. Further, as tapentadol is suitable for treating pain in patients suffering from serious diseases, e.g. for treating cancer pain, such patients including pediatric patients are usually simultaneously treated with other medicaments, e.g. chemotherapeutics, that have severe side effects. Under these circumstances, it is even more desirable to not expose such pediatric patients to preservatives, if avoidable.

In this regard, local or regional treatment is especially beneficial, since the systemic concentration of tapentadol may be kept at a sub-therapeutic level and systemic side effects that burden the entire organism may be avoided. To keep the systemic concentration of a drug at a low level is especially crucial in the treatment of pediatric patients.

A further aspect of the invention relates to a dosage form comprising an anesthetic as defined above or hereinafter in combination with tapentadol, preferably in combination with the aqueous pharmaceutical composition as described above or hereinafter.

In a preferred embodiment, the dosage form according to the invention comprises the aqueous pharmaceutical composition according to the invention.

In a preferred embodiment, the dosage form according to the invention comprises the combined pharmaceutical composition according to the invention. In this embodiment, the combined pharmaceutical composition preferably comprises the aqueous pharmaceutical composition according to the invention.

Preferably, the aqueous pharmaceutical composition according to the invention, the combination according to the invention and the dosage form according to the invention, respectively, are for use in the treatment of pain.
The pain may either be chronic pain or acute pain.

Preferably, the pain is selected from the group consisting of inflammatory pain, neuropathic pain, visceral pain, labor pain, cancer pain perioperative and post-operative pain.

In a preferred embodiment, the pain is cancer pain, preferably neuropathic pain being induced by the cancer, including neuropathic pain as a direct result of the cancer on peripheral nerves, or as a side effect of chemotherapy, surgery or radiation injury.

In another preferred embodiment, the pain is neuropathic pain associated with diabetes mellitus (diabetic polyneuropathy).

In another preferred embodiment, the pain is perioperative or post-operative (post-surgical) pain, including bunionectomy pain.

In another preferred embodiment, the pain is labor pain.

In still another preferred embodiment, the aqueous pharmaceutical composition according to the invention, the combination according to the invention and the dosage form according to the invention, respectively, are for use in emergency pain management.

In yet another preferred embodiment, the aqueous pharmaceutical composition according to the invention, the combination according to the invention and the dosage form according to the invention, respectively, are for use in the treatment of acute pain in newborn infants. Preferably, the newborn infants may have a body weight with a lower limit of 2.500 g, more preferably with a lower limit of 2.000 g, still more preferably with a lower limit of 1.500 g, yet preferably with a lower limit of 1.000 g, most preferably with a lower limit of 750 g, and in particular with a lower limit of 500 g.

The aqueous composition according to the invention and the dosage form containing the aqueous composition according to the invention, respectively, are adapted for parenteral administration of tapentadol.

The administration may proceed by infusion or injection.

Infusion solutions or suspensions may be administered continuously, intermittently or patient-controlled. For the administration, infusion devices such as implantable infusion pumps, non-implantable infusion pumps and spinal pumps may be used.

The administration of the tapentadol may proceed intramuscularly, intravenously, subcutaneously, epidurally, intrathecally, intraspinally and/or intracerebroventricularly.

In a preferred embodiment, the administration proceeds intraspinally, either intrathecally or epidurally, preferably by infusion. The intraspinal administration is especially suitable for treating pain selected from perioperative pain, post-operative pain, labor pain and cancer pain. The dosage of the intraspinal administration may be controlled by means of an infusion pump, either by the patient or by the selection of an appropriate steady or intermittent infusion rate.

In another preferred embodiment, the administration proceeds intramuscularly, intravenously or subcutaneously. This type of administration is especially preferred for the local or regional treatment of pain in distal extremities.

Depot formulations are preferably administered intramuscularly or subcutaneously.

In yet another preferred embodiment, the administration proceeds intrathecally and/or intracerebroventricularly. This type of administration is especially preferred for the treatment of neuropathic pain, including neuropathic pain associated with cancer or diabetes mellitus.

In a preferred embodiment, the administration of the tapentadol proceeds via a combination of intracerebroventricular and intrathecal administration.

Preferably, the dosage of the intrathecal administration exceeds the dosage of the intracerebroventricular administration.

In a preferred embodiment, the ratio between the dosage of tapentadol in the intrathecal administration and the dosage of tapentadol in the intracerebroventricular administration is at least 1:1, more preferably at least 1.1:1, still more preferably at least 1.2:1, yet more preferably at least 1.4:1, most preferably at least 1.6:1, and in particular at least 1.8:1.

Preferably, the combined intracerebroventricular and intrathecal administration exhibits a synergistic effect for the treatment of pain.

The skilled person is fully aware of methods to assess whether said combined administration exhibits a synergistic effect for the treatment of pain. For example, the skilled person may determine the ED 50 values for the single intrathecal administration, single intracerebroventricular administration and the combined administration, and compare the ED 50 value of the combined administration with the theoretic additive ED 50 value. Further details on this determination method are contained in the experimental section.

Preferably, the efficacy of the composition containing tapentadol according to the invention is higher than the efficacy of comparable compositions containing the same dosage of either morphin or oxycodon, respectively.

In a preferred embodiment, the pain, preferably neuropathic pain, is relieved faster after administration of the composition containing tapentadol according to the invention than after administration of a comparable composition containing the same dosage of oxycodone instead of tapentadol.

Preferably, the time period until the pain is efficiently relieved is reduced by at least 5%, more preferably at least 10%, still more preferably at least 15%, yet more preferably at least 20%, most preferably at least 25%.

In a preferred embodiment, the pain, preferably neuropathic pain, is relieved faster after administration of the composition containing tapentadol according to the invention than after administration of a comparable composition containing the same dosage of morphin instead of tapentadol.

Preferably, the time period until the pain is efficiently relieved is reduced by at least 5%, more preferably at least 10%, still more preferably at least 15%, yet more preferably at least 20%, most preferably at least 25%.

A still further aspect of the invention relates to the use of tapentadol for the manufacture of the aqueous pharmaceutical composition according to the invention as described above or of the pharmaceutical dosage form containing the aqueous pharmaceutical composition according to the invention as described above, which is adapted for parenteral administration.

A yet further aspect of the invention relates to a method for the treatment of pain comprising the parenteral administration of the aqueous pharmaceutical composition according to the invention as described above or of the pharmaceutical dosage form containing the aqueous pharmaceutical composition according to the invention as described above to a subject in need thereof.

The following examples further illustrate the invention but are not be construed as limiting its scope.

### Example 1:

a) A tapentadol HCl solution was prepared by dissolving 20 g of Tapentadol HCl in 1 L water for injection and adjusting isotonic conditions by addition of sodium chloride. The solution had a pH value of below 5.4. Thus, tapentadol HCl has slightly acidic properties when dissolved in water.
b) Tapentadol HCl solutions for injection containing 15 mg/mL of the free tapentadol base were formulated according to the following table.

**Table 1:**

| Ingredient | Content [mg / mL] |
|---|---|
| Tapentadol HCl | 17.47 |
| Sodium citrate dihydrate | 0.50 |
| Sodium chloride | 5.0 |
| Water for injections | Ad 1.003 g (1 mL) |

The formulations were spiked with *Staphylococcus aureus* (*Staph. aureus), Pseudomonas aeruginosa (Ps. aerouginosa), Aspergillus niger* (*Asp. niger*) and *Candida albicans* and their efficacy of antimicrobial preservation was evaluated according to the test "efficacy of antimicrobial preservation" as recommended by the Ph. Eur.

The test acceptance criteria for parenteral preparations according to the Ph. Eur. are given in the table 2.

The criteria A express the recommended efficacy to be achieved. In justified cases where the criteria A cannot be attained, for example for reasons of an increased risk of adverse reaction, the criteria B must be satisfied.

**Table 2: Acceptance criteria for parenteral preparations ("Efficacy of antimicrobial preservation" test, Ph. Eur.)**

| | Test criteria | Log reduction | | | | |
|---|---|---|---|---|---|---|
| | | 6 h | 24 h | 7 d | 14 d | 28 d |
| Bacteria | A | 2 | 3 | - | - | NR |
| | B | - | 1 | 3 | - | NI |
| Fungi | A | - | - | 2 | - | NI |
| | B | - | - | - | 1 | NI |

| | | | | | | |
|---|---|---|---|---|---|---|
| (NI = no increase, NR = no recover) | | | | | | |

The "efficacy of antimicrobial preservation" tests revealed that *Asp. niger* seems to be more resistant to tapentadol compared to the other three tested bacteria / fungi. Ph. Eur. test criteria A failed, whereas criteria B were passed.

### Example 2:

The effect of the inventive composition on polyneuropathical pain was then studied as follows:
Diabetical hyperalgesia was induced in male C57/BL/6 mice by a single intraperitoneal injection of a citrate buffered solution containing streptozotocin (dosage: 200 mg/kg; first injection solution). 1-2 weeks later, 5 µL of a second injection solution containing tapentadol or vehicle was injected intrathecally, 5 µl of a third injection containing vehicle was injected intracerebroventricularly and the mouse was placed on a hot plate maintained at 50 °C and the number of nocifensive reactions (licking/shaking of the hindlimbs, licking of the genitals, jumping) was recorded. The cut off time was set at 2 minutes.

Thermal hyperalgesia thresholds (withdrawal latency) were measured at short time intervals directly after the injection (after 15, 30, 45 and 60 min). The group size was 10. The antihyperalgesic activity of the tested pharmaceutical composition is expressed as percentages of maximum possible effect (%MPE).

By means of said test, the antihyperalgesic activity of the composition according to the invention was studied for three different dosages (0.316 µg, 1.00 µg and 3.16 µg). For comparison, two comparative experiments were also conducted.

The different conditions are summarized in the following table:

**Table 3:**

| | I-1 | I-2 | I-3 | C-1 | C-2 |
|---|---|---|---|---|---|
| 1. injection solution: citrate solution containing | STZ | STZ | STZ | STZ | - |
| 2. injection solution: dosage of tapentadol [µg] | 0.316 | 1.00 | 3.16 | - | - |
| 3. injection solution: vehicle solution | - | - | - | - | - |

| | | | | | |
|---|---|---|---|---|---|
| STZ: streptozotocin | | | | | |

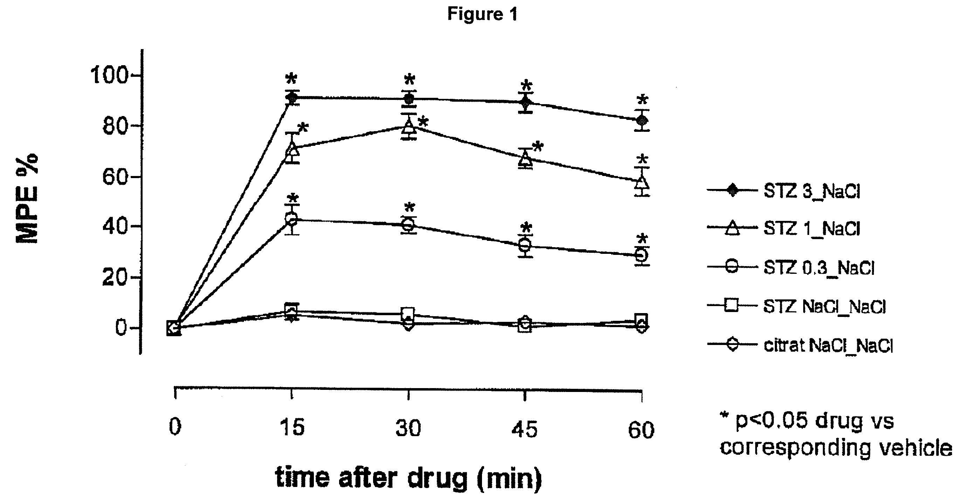

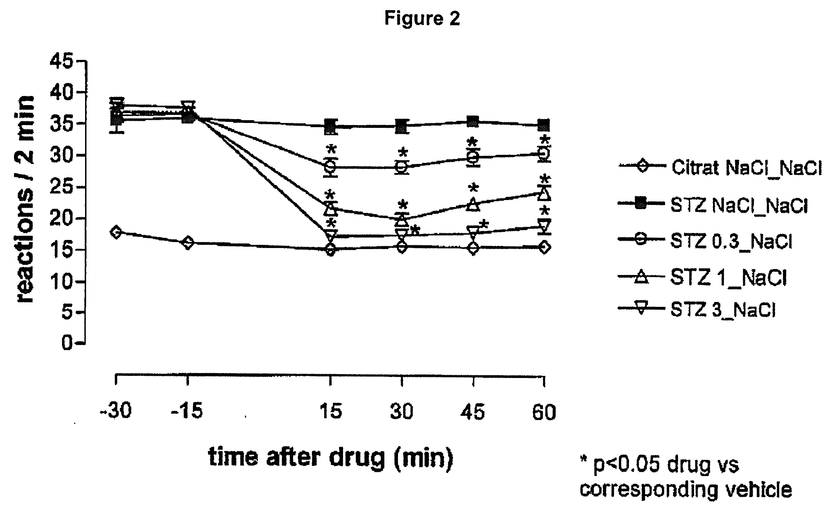

The results concerning the antihyperalgesic activities are depicted in Figure 1. The absolute numbers of withdrawals are depicted in Figure 2 for each experiment.

It becomes evident from Figures 1 and 2, that tapentadol exhibits dose-dependent inhibition of diabetic heat hyperalgesia. Using the thermal hyperalgesia threshold values after 15 minutes, the ED-50 value was calculated to be 0.42 (0.26-0.58) mg/animal for the intrathecal administration.

### Example 3:

According to Example 2, but with the variation that the second injection solution was injected intracerebroventricularly and the third injection solution containing vehicle was injected intrathecally, the effect of the inventive composition on polyneuropathical pain was studied using the following injection solutions:

**Table 4:**

| | I-2 | I-4 | I-5 | C-1 | C-2 |
|---|---|---|---|---|---|
| 1. injection solution: citrate solution containing | STZ | STZ | STZ | STZ | - |
| 2. injection solution: dosage of tapentadol [µg] | 1.00 | 0.3 | 0.1 | - | - |
| 3. injection solution: vehicel | - | - | - | - | - |

| | | | | | |
|---|---|---|---|---|---|
| STZ: streptozotocin | | | | | |

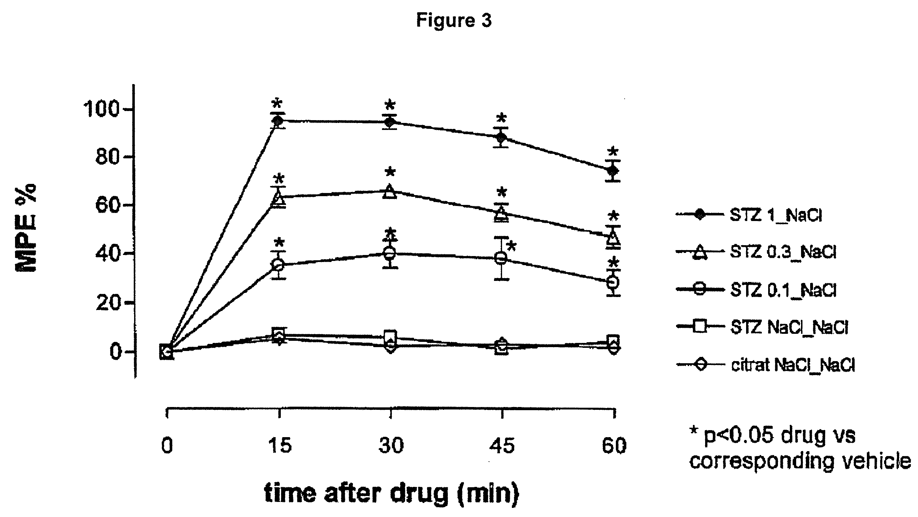

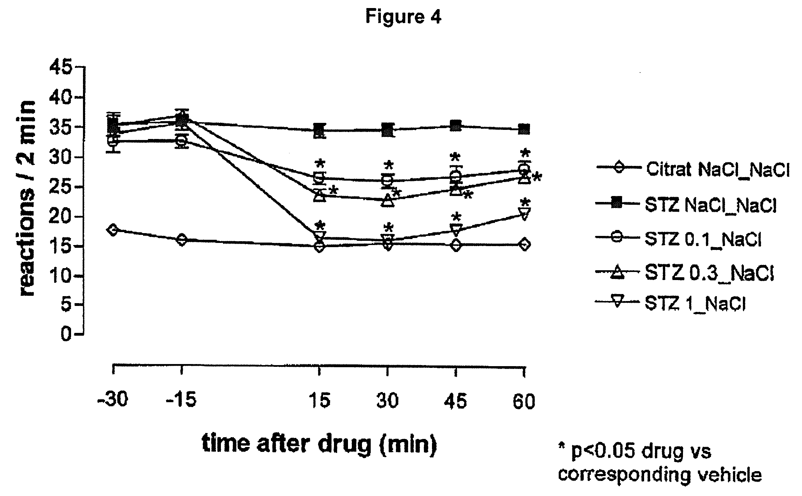

The results concerning the antihyperalgesic activities are depicted in Figure 3. The absolute numbers of withdrawals are depicted in Figure 4 for each experiment.

It becomes evident from Figures 3 and 4, that tapentadol exhibits dose-dependent inhibition of diabetic heat hyperalgesia. Using the thermal hyperalgesia threshold values after 15 minutes, the ED-50 value was calculated to be 0.18 (0.14-0.22) mg/animal for the intracerebroventricular administration.

### Example 4:

According to Example 1, the effect of the inventive composition on polyneuropathical pain was studied, but with the variation that two injection solutions containing tapentadol or vehicle were administered simultaneously: one intrathecally (2. injection solution) and another one intracerebroventricularly (3. injection solution). The following injection solutions were used:

**Table 5:**

| | I-6 | I-7 | I-8 | C-3 | C-4 |
|---|---|---|---|---|---|
| 1. injection solution: citrate solution containing | STZ | STZ | STZ | STZ | - |
| dosage of tapentadol [µg] | | | | | |
| 2. injection solution | 0.2 | 0.06 | 0.02 | - | - |
| 3. injection solution | 0.1 | 0.03 | 0.01 | - | - |

| | | | | | |
|---|---|---|---|---|---|
| STZ: streptozotocin | | | | | |

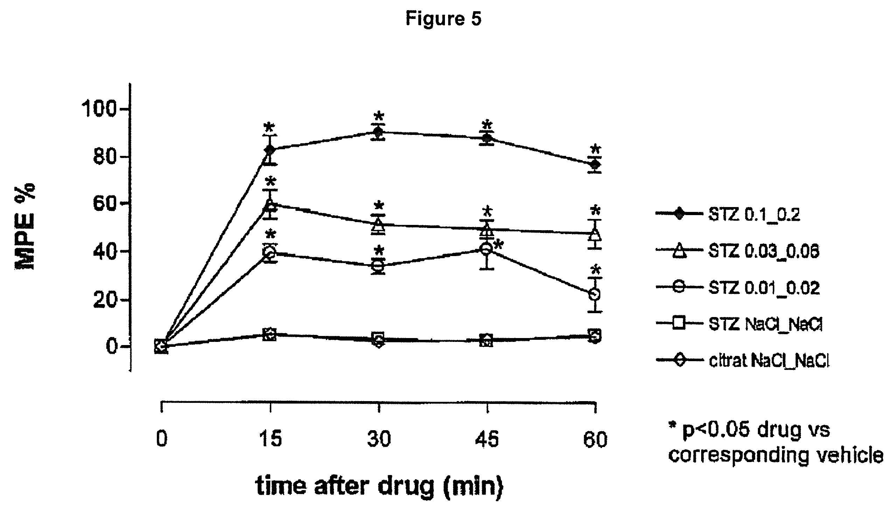

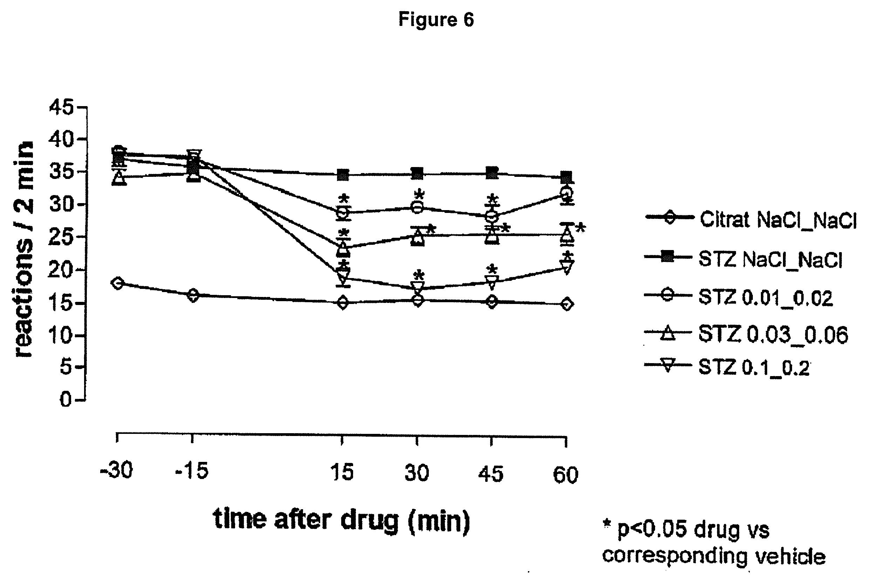

The results concerning the antihyperalgesic activities are depicted in Figure 5. The absolute numbers of withdrawals are depicted in Figure 6 for each experiment.

It becomes evident from Figures 5 and 6, that tapentadol exhibits dose-dependent inhibition of diabetic heat hyperalgesia. Using the thermal hyperalgesia threshold values after 15 minutes, the ED-50 value was calculated to be 0.053 (0.032-0.074) mg/animal for the combination of intrathecal and intracerebroventricular administration.

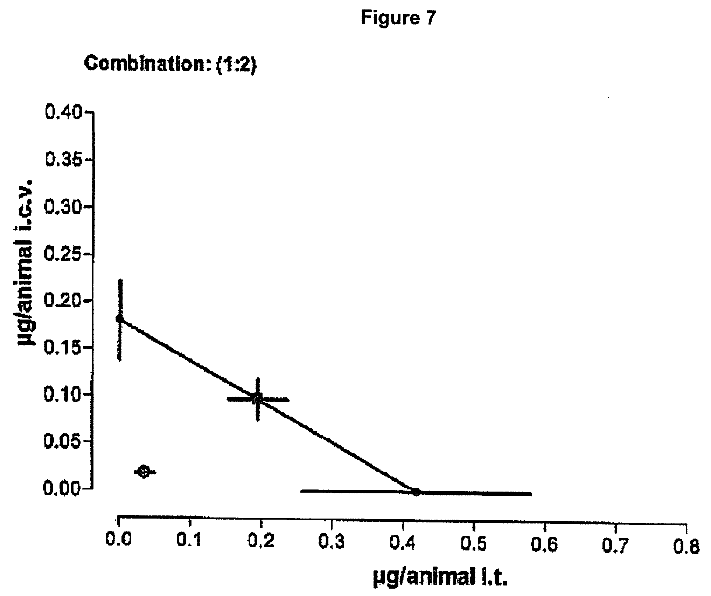

In Figure 7 the calculated ED 50 value of combined intrathecal and intracerebroventricular administration is compared to the theoretical additive effects as calculated based on the ED50 values of the intrathecal administration and of the intracerebroventricular administration. It becomes evident, that the combination of intrathecal and intracerebroventricular administration is synergistically better (p < 0.001).

### Example 5:

### Pharmacological methods:

The weight ratios of the components (a) and (b) that will lead to a supra-additive effect (synergistic effect) of the inventive pharmaceutical composition may be determined via the paw pressure test of Randall and Selitto as described in Arch. Int. Pharmacodyn., 1957, 111: 409 to 419, which is a model for inflammatory pain. The respective part of the literature is hereby incorporated by reference and forms part of the present disclosure.

Accordingly, the weight ratios of the components (a) and (b) that will lead to a supra-additive effect (synergistic effect) of the inventive pharmaceutical composition were determined in a model of acute pain (A) and a model of chronic pain (B).

### A) Carrageenan induced acute inflammatory pain (Paw pressure test) in rats

Acute inflammation was induced by injection of a 0.1 ml carrageenan solution (0.5 % in distilled water) subcutaneously into the plantar surface of the right hind paw of the rat. The mechanical nociceptive threshold was measured using an Algesiometer (Ugo Basile, Italy). The device generates a mechanical force with a linear increase over time. The force was applied to the dorsal surface of the inflamed hind paw via a cone-shaped stylus with a rounded tip (3 mm²). The nociceptive threshold (T_{V}) was defined as the force at which the rat vocalises (cut-off force 450 g). Compounds or vehicle were given 3 h after carrageenan injection. The mechanical nociceptive threshold was measured at different times after the drug or vehicle administration. The drug effects are expressed as percentages of the maximal possible effect (MPE) based on the following formula: %MPE = (T_{V drug} - T_{V control}) / (cut-off - T_{V control}) x 100. The group size is n = 12.

### B) Complete Freund Adjuvant (CFA) induced chronic inflammatory pain (Paw pressure test) in rats

Chronic inflammation was induced by an intraplantar injection of 0.05 mL of a CFA solution (Mycobacterium tuberculosis, H37 Ra, Difco Laboratories, Detroit, Mi., U.S.A.; 1mg/mL in mineral oil) into one hind paw. The mechanical nociceptive threshold is measured before (prevalue) and one day after CFA injection using an Algesiometer (Ugo Basile, Italy). The device generates a mechanical force with a linear increase over time. The force is applied to the dorsal surface of the inflamed rat hind paw via a cone-shaped stylus with a rounded tip (2 mm tip diameter). The nociceptive threshold is defined as the force (in grams) at which the rat vocalises (cut-off force 450 g). The mechanical nociceptive threshold is measured at different timepoints after the drug or vehicle administration. The antinociceptive and antihyperalgesic activity of the tested substance is expressed as percentages of the maximal possible effect (%MPE). The maximal possible effect is defined as the percentage of prevalue withdrawal treshold before CFA-injection. The group size is n = 10.

### Analysis of results in Carrageenan induced (acute) and CFA-induced (chronic) inflammatory pain

The analysis of the results with respect to a supra-additive effect of the inventive pharmaceutical composition comprising the components (a) and (b) was carried out via statistical comparison of the theoretical additive ED₅₀-value with the experimentally determined ED₅₀-value of a so-called fixed ratio combination (isobolographic analysis according to Tallarida JT, Porreca F, and Cowan A. Statistical analysis of drug-drug and site-site interactions with isobolograms. Life Sci 1989; 45: 947 - 961).

The interactions studies presented herein were performed using equieffective doses of the two components, calculated from the ratio of the respective ED₅₀ values of the components if administered alone.

### Results

The application route was intravenous (i.v.) and intraperitoneal (i.p.) for tapentadol (A) ((-)-(1R,2R)-3-(3-dimethy)amino-1-ethy)-2-methyl-propyl)-phenol) in Carrageenan- and CFA-induced inflammatory pain, respectively, and intraperitoneal (i.p.) for lidocain (lidocain hydrochloride) in both animal models. The peak effect of tapentadol (A) in the Carrageenan-induced (acute) inflammatory pain model was reached 15 min p. appl. (timepoint of first measurement)] and ED₅₀-value of 1.75 (1.69 -1.81) mg/kg i.v. was calculated. The peak effect of tapentadol (A) in the CFA-induced (chronic) inflammatory pain model was reached 15 min p. appl. (timepoint of first measurement)] and ED₅₀-value of 6.34 (4.42 - 8.08) mg/kg i.p. was calculated. Lidocain induced dose-dependent analgesic effects with an ED₅₀-value of 32.4 (30.0 - 34.6) mg/kg i.p. in the Carrageenan-induced (acute) inflammatory pain model, and an ED₅₀-value of 26.8 (25.2 - 29.0) mg/kg i.p., in the CFA-induced (chronic) inflammatory pain model, reaching the peak effect 15 min p. appl. in both models. According to their respective timepoint of peak effect, tapentadol (A) was applied 15 min and lidocain 15 min before timepoint of measurement of the interaction-experiments (i. e. both components were applied simultaneously) in both animal models.

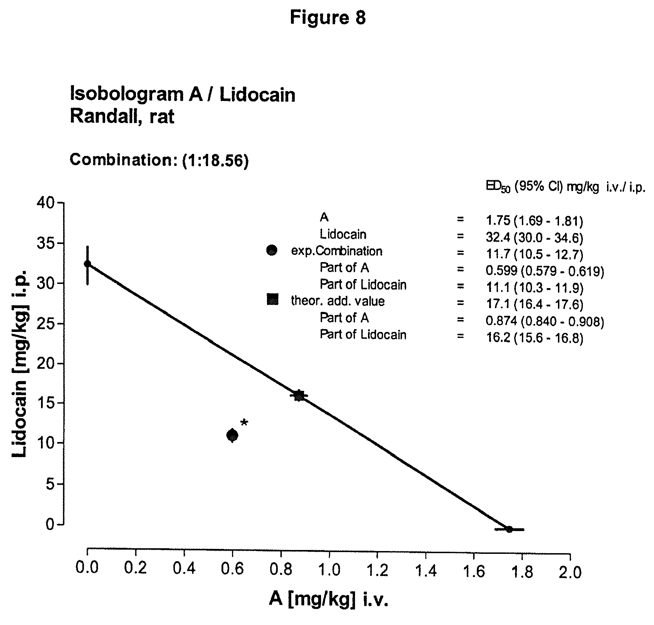

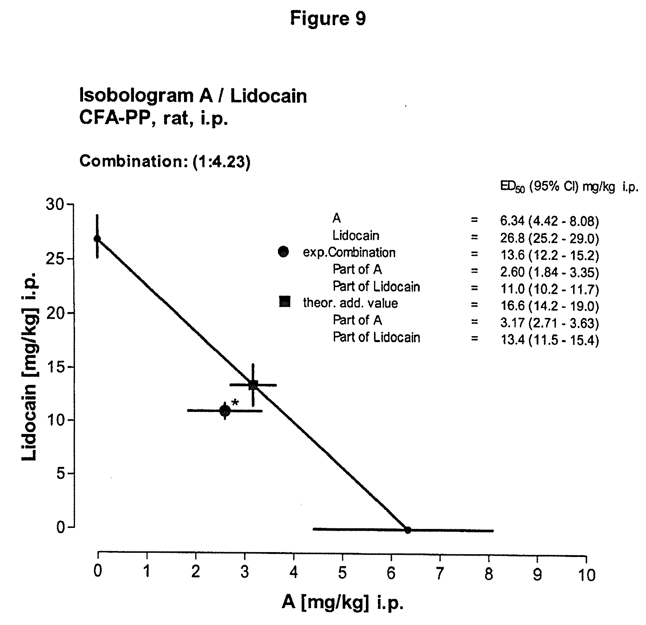

Thus, the time point of ED₅₀ calculation of the combination of A with Lidocain corresponds to the timepoint of the peak effect of the respective compound. The isobolographic analysis revealed that the experimental ED₅₀-values of the combinations in the Carrageenan- (Figure 8) as well as in the CFA-induced inflammatory pain model (Figure 9), were significantly lower than the respective theoretical ED₅₀-values. Thus, the combination studies demonstrate synergistic interaction of tapentadol (A) with the Licocain in an acute and chronic animal model for inflammatory pain.

The results of the isobolographic analysis are summarized in the following table.

**Table 6: Experimental ED₅₀ values of tapentadol (A) and lidocain and isobolographic analysis of the interaction between tapentadol (A) with lidocain.**

| Pain model | Substance / ED₅₀ [mg/kg] | Tapentadol (A) | Lidocain | Theoretical ED₅₀ of the combination | Experimental ED₅₀ of combination | Interaction |
|---|---|---|---|---|---|---|
| acute pain | tapentadol (A) + lidocain | 1.75 (1.69-1.81) | 32.4 (30.0 -34.6) | 17.1 (16.4-17.6) | 11.7 (10.5-12.7) | supra-additive (p<0.001) |
| chronic pain | tapentadol (A) + lidocain | 6.34 (4.42-8.08) | 26.8 (25.2 -29.0) | 16.6 (14.2 - 19.0) | 13.6 (12.2-15.2) | supra-additive (p<0.01) |

| | | | | | | |
|---|---|---|---|---|---|---|
| p: level of statistical significance of supra-additive interaction | | | | | | |

The ratios of tapentadol with + lidocain used in the aforementioned experiments are summarized in the table here below.

**Table 7:**

| Animal pain model | Combination of tapentadol (A) with | Ratio |
|---|---|---|
| acute pain | lidocain | 1: 18.56 |
| chronic pain | lidocain | 1 : 4.23 |

### Example 6:

### Antimicrobial effect of tapentadol at pH 3 and pH 8

A tapentadol solution with a concentration of 15 mg/mL tapentadol (free base) was prepared. The pH-value was adjusted to the target value of 3 or 8 using citric acid and 1N NaOH solution, respectively. No additional buffer system was added. To ensure the placebo solution shows no antimicrobial effect itself, a placebo solution pH 8 was prepared, with focus on the same pH-value, even though a different amount of 1 N NaOH solution was used for pH adjustment.

The formulations were prepared, filled in glass bottles and sterilized in an autoclave for 30 min at 121°C and 2 bars. The sterilized glass bottles were spiked with *Staphylococcus aureus* (*Staph. aureus), Pseudomonas aeruginosa (Ps. aerouginosa), Aspergillus niger (Asp. niger)* and *Candida albicans* for the test "Efficacy of antimicrobial preservation" on the basis of Ph. Eur. 6.6 monograph 5.1.3.

The Ph. Eur. test acceptance criteria for parenteral preparations are given in Table (NI = no increase, NR = no recover). The A criteria express the recommended efficacy to be achieved, in justified cases where the A criteria cannot be attained for example for reasons of an increased risk of adverse reaction, the B criteria must be satisfied. To reduce the amount of experiments for this first set up of pH-value experiments, the test points at 6 and 24 hours were replaced by a test point at 30 min (table 8).

**Table 8 Acceptance criteria for parenteral preparations for "Efficacy of antimicrobial preservation" (Ph. Eur.)**

| Log reduction | | | | | | |
|---|---|---|---|---|---|---|
| | Test criteria | 6 h | 24 h | 7 d | 14 d | 28 d |
| Bacteria | A | 2 | 3 | - | - | NR |
| | B | - | 1 | 3 | - | NI |
| Fungi | A | - | - | 2 | - | NI |
| | B | - | - | - | 1 | NI |

The results for the microbial testing of the solutions are given for each bacteria / fungi in Tables 9 to 12.

**Table 9 Microbial growth of Staph. aureus**

| Microbial count | Placebo pH 8 | Tapentadol pH 8 | Tapentadol pH 3 |
|---|---|---|---|
| Spiked amount of bacteria /fungi | 7.4 x 10³ | 1.7 x 10⁶ | 1.6 x 10⁶ |
| 30 min | 8.3 x 10⁵ | 8 x 10⁵ | 2.5 x 10⁶ |
| 7 days | 2.8 x 10⁵ | < x 10² | 2.3 x 10³ |
| 14 days | not tested | < x 10² | < x 10² |
| 28 days | not tested | < x 10² | < x 10¹ |
| Test criteria A | failed | passed | passed |
| Test criteria B | failed | passed | passed |

**Table 10 Microbial growth of Ps. aeruginosa**

| Microbial count | Placebo pH 8 | Tapentadol pH 8 | Tapentadol pH 3 |
|---|---|---|---|
| Spiked amount of bacteria /fungi | 1.4 x 10⁶ | 1.7 x 10⁶ | 1.6 x 10⁶ |
| 30 min | 1.6 x 10⁶ | < x 10⁴ | 4.5 x 10⁵ |
| 7 days | 8.8 x 10⁶ | < x 10² | 2 x 10³ |
| 14 days | not tested | < x 10² | < x 10² |
| 28 days | not tested | < x 10² | < x 10² |
| Test criteria A | failed | passed | passed |
| Test criteria B | failed | passed | passed |

**Table 11 Microbial growth of Asp. niger**

| Microbial count | Placebo pH 8 | Tapentadol pH 8 | Tapentadol pH 3 |
|---|---|---|---|
| Spiked amount of bacteria /fungi | 4.2 x 10⁵ | 5.4 x 10⁵ | 3.9 x 10⁵ |
| 30 min | 4.3 x 10⁵ | 6 x 10⁵ | 4.5 x 10⁵ |
| 7 days | 6.3 x 10⁵ | 4.5 x 10² | 8 x 10⁴ |
| 14 days | not tested | 0.3 x 10² | 4.1 x 10⁵ |
| 28 days | not tested | 1.8 x 10¹ | 4.5 x 10⁵ |
| Test criteria A | failed | passed | failed |
| Test criteria B | failed | passed | failed |

**Table 12 Microbial growth of Candida albicans**

| Microbial count | Placebo pH 8 | Tapentadol pH 8 | Tapentadol pH 3 |
|---|---|---|---|
| Spiked amount of bacteria /fungi | 2 x 10⁵ | 1.7 x 10⁵ | 2.4 x 10⁵ |
| 30 min | 2.5 x 10⁵ | < x 10⁴ | 2 x 10⁵ |
| 7 days | 3.4 x 10⁶ | < x 10² | 1.3 x 10³ |
| 14 days | not tested | < x 10² | 1.8 x 10³ |
| 28 days | not tested | < x 10² | 2.5 x 10³ |
| Test criteria A | failed | passed | failed |
| Test criteria B | failed | passed | failed |

In the absence of additional preservatives, the tapentadol solution pH 3 is not sufficiently preserved according to Ph. Eur. (crit. A and B) for *Asp. niger* and *Cand. albicans,* whereas the tapentadol solution pH 8 passed the crit. A and B for all tested bacteria and funghi. The placebo pH 8 solution shows no preservative effect of the solution itself, so that the antimicrobial effect of the formulation containing tapentadol HCl is a consequence of the added amount of tapentadol HCl. Considering this results a clear dependency of the pH-value on the preserving effect of the tapentadol HCl solution could be shown.

The tapentadol HCl solution with a higher pH value of 8 has an improved antimicrobial effect compared to the pH 3 solution, so a clear dependency of the pH - value of the solution on the preserving effect of tapentadol was found.

## Claims

1. An aqueous pharmaceutical composition adapted for parenteral administration of tapentadol or a physiologically acceptable salt thereof having a pH value of at least 5.4.

2. The composition according to claim 1, which is adapted for local and/or systemic administration.

3. The composition according to claim 1 or 2, which is adapted for administration by injection or infusion.

4. The composition according to any of the preceding claims, wherein the concentration of tapentadol is below 50 mg/mL, based on the total volume of the composition.

5. The composition according to any of the preceding claims, which additionally contains a buffer.

6. The composition according to any of the preceding claims, which does not contain any preservative.

7. The composition according to any of the preceding claims, which has an osmolarity of at least 0.25 osmol/L.

8. The composition according to any of the preceding claims, which is adapted for administration in combination with an anesthetic.

9. A pharmaceutical dosage form comprising the pharmaceutical composition according to any of the preceding claims.

10. The dosage form according to claim 9, which is adapted for administration to pediatrics.

11. The dosage form according to claim 9 or 10, which is a depot formulation.

12. The composition according to any of claims 1 to 8 or the dosage form according to any of claims 9 to 11 for use in the treatment of pain.

13. The composition according to claim 12, wherein the pain is selected from the group consisting of diabetic neuropathic pain, cancer pain, perioperative and/or post-operative pain.

14. The composition according to claim 12 or 13, wherein administration proceeds intramuscularly, intravenously, subcutaneously, epidurally, intrathecally, intraspinally and/or intracerebroventricularly.

15. The composition according to any of claims 12 to 14, wherein the dosage of tapentadol to be administered is adjusted by the patient.
